(19) Europäisches Patentamt
European Patent Office
Office européen des brevets

(11) Veröffentlichungsnummer: **0 087 048**
**B1**

(12) **EUROPÄISCHE PATENTSCHRIFT**

(45) Veröffentlichungstag der Patentschrift: **17.04.85**

(21) Anmeldenummer: **83101122.6**

(22) Anmeldetag: **07.02.83**

(51) Int. Cl.[4]: **C 07 C 131/00**, A 01 N 37/46, C 07 D 317/28, C 07 D 231/12, C 07 D 249/08, C 07 D 307/32, C 07 D 319/06, C 07 D 487/02, A 01 N 43/28, A 01 N 47/34, A 01 N 43/56

(54) **Substituierte Oximinoacetanilide, Verfahren zu ihrer Herstellung sowie ihre Verwendung als Schädlingsbekämpfungsmittel.**

(30) Priorität: **20.02.82 DE 3206235**

(43) Veröffentlichungstag der Anmeldung: **31.08.83 Patentblatt 83/35**

(45) Bekanntmachung des Hinweises auf die Patenterteilung: **17.04.85 Patentblatt 85/16**

(84) Benannte Vertragsstaaten: **AT BE CH DE FR GB IT LI NL SE**

(56) Entgegenhaltungen:
**EP - A - 0 000 023**
**EP - A - 0 011 047**
**EP - A - 0 020 859**
**BE - A - 870 067**
**DE - A - 2 312 956**

(73) Patentinhaber: **BAYER AG, Konzernverwaltung RP Patentabteilung, D-5090 Leverkusen 1 Bayerwerk (DE)**

(72) Erfinder: **Brandes, Wilhelm, Dr., Eichendorffstrasse 3, D-5653 Leichlingen (DE)**
Erfinder: **Daum, Werner, Dr., Bärenstrasse 18, D-4150 Krefeld (DE)**

Anmerkung. Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 087 048 B1

BUNDESDRUCKEREI BERLIN

**Beschreibung**

Die vorliegende Erfindung betrifft neue substituierte Oximinoacetanilide, Verfahren zu ihrer Herstellung sowie ihre Verwendung als Schädlingsbekämpfungsmittel, insbesondere als Fungizide.

Wie bereits lange bekannt ist, werden im Pflanzenschutz Fungizide in der Landwirtschaft und im Gartenbau, insbesondere das Zink-ethylen-1,2-bis-dithiocarbamidat und das N-Trichlormethylthio-tetrahydrophthalimid, verwendet; die genannten Verbindungen besitzen unter den Handelsprodukten eine große Bedeutung (vgl. R. Wegler, »Chemie der Pflanzenschutz- und Schädlingsbekämpfungsmittel«, Band 2, Seiten 65 und 108, Berlin/Heidelberg/New York (1970)). Die Wirkung bei niedrigen Aufwandkonzentrationen ist jedoch nicht immer ganz befriedigend.

Weiterhin ist bekannt, daß einige Isonitroso-cyanacetamid-Derivate (DE-OS 2 312 956, US-PS 3 919 284, und 3 957 847 und Alkoxycarbonylethyl-N-haloacetylaniline (DE-OS 2 350 944) fungizide Eigenschaften besitzen. Auch hier ist die Wirksamkeit bei niedrigen Aufwandmengen nicht immer befriedigend und bei höheren Konzentrationen können Pflanzenschäden auftreten.

Weiterhin sind omega-substituierte Pentylharnstoff-Derivate, wie z.B. 1-(5-Cyanpentyl)-3-[2-cyan-2-(2-ethylphenylamino-carbonylmethyl-oximino-acetyl)]-harnstoff und ihre Verwendung als Fungizide bekannt (vgl. EP-A 0 000 023).

Die Wirksamkeit ist bei niedrigen Aufwandmengen bzw. -konzentrationen nicht immer voll befriedigend.

Es wurden neue substituierte Oximinoacetanilide der Formel (I) gefunden

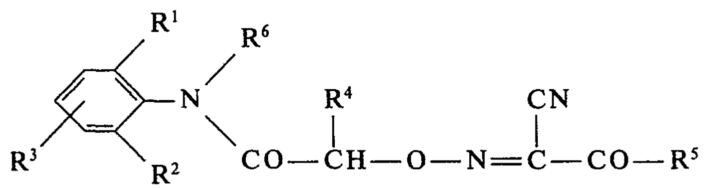

(I)

in welcher

$R^1$ Alkyl, Alkoxy oder Halogen,
$R^2$ Wasserstoff, Alkyl, Halogen oder Trifluormethyl,
$R^3$ Wasserstoff, Alkyl oder Halogen,
$R^4$ Wasserstoff oder Methyl,
$R^5$ Alkoxy, Amino oder $-NH-CO-NH-R^7$ bedeutet, wobei $R^7$ für Wasserstoff oder gegebenenfalls durch Cyan-, Alkoxy- oder Alkoxycarbonyl-substituiertes Alkyl bzw. für Cycloalkyl steht und
$R^6$ Alkinyl, Alkoxyalkyl, Halogenalkoxyalkyl, Cyanalkoxyalkyl, Cycloalkoxyalkyl, Alkenoxyalkyl, Alkinoxyalkyl, Alkylthioalkyl, Alkylcarbonylalkyl, Alkoxycarbonylalkyl, Alkylthiocarbonylalkyl bedeutet, außerdem Aminocarbonylalkyl, wobei die Aminogruppe durch Alkylreste substituiert sein kann, die auch zusammen mit dem Stickstoffatom einen heterocyclischen Ring bilden können, weiterhin ein 5- oder 6-gliedriger über C- oder N-verknüpfter Heterocyclylalkyl-Rest, der bis zu 3 Stickstoffatomen, bis zu 2 Sauerstoffatomen oder bis zu 2 Stickstoff- und 1 Sauerstoffatom enthalten kann, einen Bicycloheterocyclylalkyl-Rest mit bis zu 3 Stickstoffatomen, wobei die heterocyclischen Reste durch Alkyl- oder die Ketogruppe weiter substituiert sein können, ein hydrierter Furanon-Rest, der durch Alkylgruppen substituiert sein kann oder ein Rest der Struktur

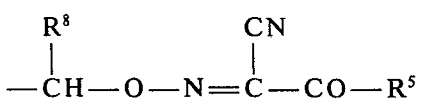

bedeutet, wobei
$R^5$ die obige Bedeutung hat und
$R^8$ für Wasserstoff, Methyl oder Ethyl steht.

Die erfindungsgemäßen substituierten Oximinoacetanilide der allgemeinen Formel (I) können in verschiedenen stereochemischen Formen vorliegen. Formel (I) umfaßt alle möglichen Formen und deren Gemische.

Man erhält die substituierten Oximinoacetanilide der Formel (I)

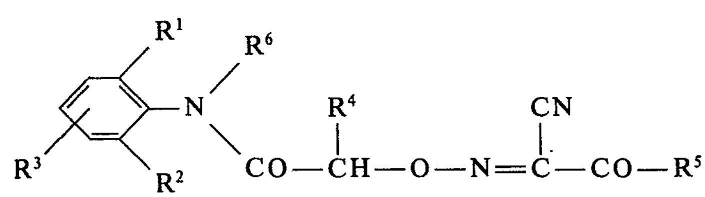

(I)

in welcher

$R^1$ Alkyl, Alkoxy oder Halogen,
$R^2$ Wasserstoff, Alkyl, Halogen oder Trifluormethyl,
$R^3$ Wasserstoff, Alkyl oder Halogen,
$R^4$ Wasserstoff oder Methyl,
$R^5$ Alkoxy, Amino oder $-NH-CO-NH-R^7$ bedeutet, wobei $R^7$ für Wasserstoff oder gegebenenfalls durch Cyan-, Alkoxy- oder Alkoxycarbonyl-substituiertes Alkyl oder für Cycloalkyl steht und
$R^6$ Alkinyl, Alkoxyalkyl, Halogenalkoxyalkyl, Cyanalkoxyalkyl, Cycloalkoxyalkyl, Alkenoxyalkyl, Alkinoxyalkyl, Alkylthioalkyl, Alkylcarbonylalkyl, Alkoxycarbonylalkyl, Alkylthiocarbonylalkyl bedeutet, außerdem Aminocarbonylalkyl, wobei die Aminogruppe durch Alkylreste substituiert sein kann, die auch zusammen mit dem Stickstoffatom einen heterocyclischen Ring bilden können, weiterhin ein 5- oder 6-gliedriger über C- oder N-verknüpfter Heterocyclylalkyl-Rest, der bis zu 3 Stickstoffatomen, bis zu 2 Sauerstoffatomen oder bis zu 2 Stickstoff- und 1 Sauerstoffatom enthalten kann, einen Bicycloheterocyclylalkyl-Rest mit bis zu 3 Stickstoffatomen, wobei die heterocyclischen Reste durch Alkyl- oder die Ketogruppe weiter substituiert sein können, ein hydrierter Furanon-Rest, der durch Alkylgruppen substituiert sein kann oder ein Rest der Struktur

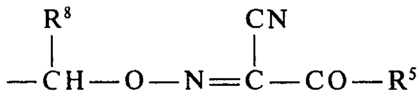

bedeutet, wobei
$R^5$ die obige Bedeutung hat und
$R^8$ für Wasserstoff, Methyl oder Ethyl steht,

wenn man

a) ein substituiertes Acetanilid der Formel (II)

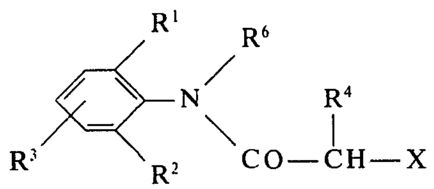

(II)

in welcher

$R^1$, $R^2$, $R^3$, $R^4$ und $R^6$ die obigen Bedeutungen haben und
X für Chlor, Brom, Jod oder für einen Sulfonyloxyrest steht,

mit einem 2-Cyan-2-oximinoessigsäurederivat der Formel (III)

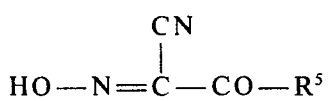

(III)

in welcher

$R^5$ die obige Bedeutung besitzt, gegebenenfalls in einem Verdünnungsmittel in Form der Alkali- oder Erdalkalisalze oder in Gegenwart eines Protonenakzeptors umsetzt, oder

wenn man

b) für den Fall, daß $R^5$ für $-NH-CO-NHR^9$ steht und

$R^9$ für einen gegebenenfalls durch Cyan-, Alkoxy- oder Alkoxycarbonyl-substituierten Alkyl- oder für einen Cycloalkyl-Rest steht, aber
$R^6$ nicht für

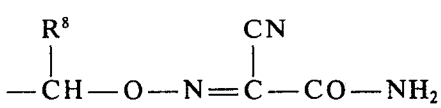

bzw.

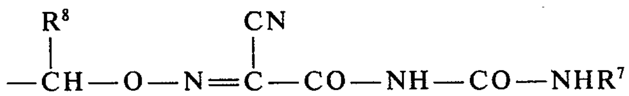

steht,

Verbindungen der Formel (IV)

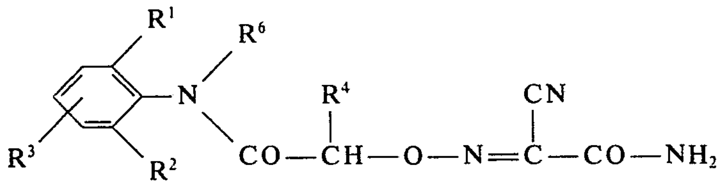

(IV)

in welcher

$R^1$, $R^2$, $R^3$ und $R^4$ die obigen Bedeutungen haben,
$R^6$ die obengenannte Bedeutung hat, aber nicht für

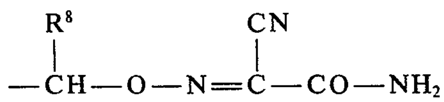

bzw.

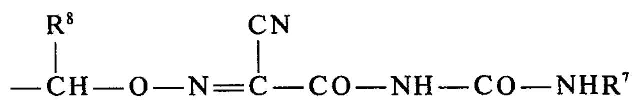

steht,

in Gegenwart einer Base mit einem Isocyanat der Formel (V)

$$OCN{-}R^9 \qquad (V)$$

in welcher

$R^9$ die obige Bedeutung hat, gegebenenfalls in Gegenwart eines Verdünnungsmittels umsetzt, oder

c) für den Fall, daß $R^6$ für

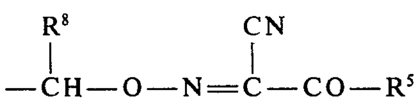

steht und $R^5$ in beiden Resten identisch ist, Verbindungen der Formel (VI)

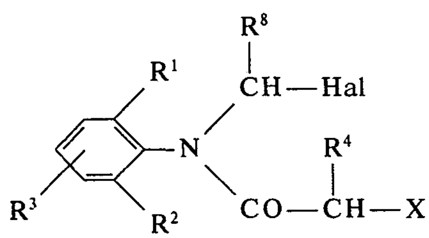

(VI)

in welcher

$R^1$, $R^2$, $R^3$, $R^4$, $R^8$ und X die obigen Bedeutungen haben und
Hal für Halogen, vorzugsweise Chlor steht,

mit 2 Mol eines 2-Cyan-2-oximinoessigsäurederivats der Formel (III) gegebenenfalls in einem Verdünnungsmittel in Form der Alkali- oder Erdalkalisalze oder in Gegenwart eines Protonenakzeptors umsetzt.

Die neuen substituierten Oximinoacetanilide der Formel (I) weisen starke fungizide Eigenschaften auf. Sie sind protektiv, curativ und eradikativ anwendbar. Außerdem haben sie systemische und/oder locosystemische Eigenschaften. Überraschenderweise zeigen sie eine bessere Pflanzenverträglichkeit als die bekannten Isonitrosocyanacetamid-Derivate. Gegenüber den Dithiocarbamidaten und dem N-Trichlormethylthio-tetrahydrophthalimid besitzen sie den Vorteil curativer und eradikativer Wirkung.

Die erfindungsgemäßen Verbindungen stellen wegen der vielen Möglichkeiten ihrer überlegenen biologischen Anwendung eine wertvolle Bereicherung der Technik dar.

Ein weiterer wesentlicher Gesichtspunkt dieser Erfindung ist, daß neue Wirkstoffe mit für die Praxis wertvollen Eigenschaften zu einer Zeit zur Verfügung gestellt werden, da durch Resistenzerscheinungen ältere Wirkstoffe für den Markt ausfallen.

Es besteht daher heute und in absehbarer Zeit ein ausgesprochener Bedarf nach neuen Fungiziden.

Von den erfindungsgemäßen substituierten Oximinoacetaniliden der Formel (I) sind diejenigen bevorzugt, bei denen

$R^1$ $C_1$—$C_4$-Alkyl, $C_1$—$C_4$-Alkoxy oder Halogen,
$R^2$ Wasserstoff, $C_1$—$C_4$-Alkyl, Trifluormethyl oder Halogen,
$R^3$ Wasserstoff, Methyl oder Halogen,
$R^4$ Wasserstoff oder Methyl,
$R^5$ $C_1$—$C_4$-Alkoxy, Amino oder —NH—CO—NH—$R^7$ bedeutet, wobei $R^7$ für Wasserstoff, gegebenenfalls durch Cyan-, $C_1$—$C_4$-Alkoxy- oder $C_1$—$C_4$-Alkoxycarbonyl-substituiertes $C_1$—$C_5$-Alkyl bzw. für $C_3$—$C_6$-Cycloalkyl steht und
$R^6$ $C_3$—$C_5$-Alkinyl, $C_1$—$C_4$-Alkoxy-$C_1$—$C_3$-alkyl, Halogen-$C_1$—$C_4$-alkoxy-$C_1$—$C_3$-alkyl, Cyan-$C_1$—$C_4$-alkoxy-$C_1$—$C_3$-alkyl, $C_4$—$C_7$-Cycloalkoxy-$C_1$—$C_3$-alkyl, $C_2$—$C_4$-Alkenoxy-$C_1$—$C_3$-alkyl, $C_2$—$C_4$-Alkinoxy-$C_1$—$C_3$-alkyl, $C_1$—$C_4$-Alkylthio-$C_1$—$C_3$-alkyl, $C_1$—$C_4$-Alkylcarbonyl-$C_1$—$C_3$-alkyl, $C_1$—$C_4$-Alkoxycarbonyl-$C_1$—$C_3$-alkyl, $C_1$—$C_4$-Alkylthiocarbonyl-$C_1$—$C_3$-alkyl bedeutet, außerdem Aminocarbonyl-$C_1$—$C_3$-alkyl, wobei die Aminogruppe durch 1 bis 2 $C_1$—$C_3$-Alkylreste substituiert sein kann, die auch zusammen mit dem Stickstoffatom einen heterocyclischen Ring bilden können, ferner ein 5- oder 6-gliedriger über C- oder N-verknüpfter Heterocyclyl-$C_1$—$C_4$-alkyl-Rest, der bis zu 3 Stickstoffatomen, bis zu 2 Sauerstoffatomen oder bis zu 2 Stickstoff- und 1 Sauerstoffatom enthalten kann, einen Bicycloheterocyclylalkyl-Rest mit bis zu 3 Stickstoffatomen, wobei die heterocyclischen Reste durch $C_1$—$C_2$-Alkyl- oder eine Ketogruppe substituiert sein können, ein hydrierter Furanon-Rest, der durch Alkylgruppen substituiert sein kann oder ein Rest der Struktur

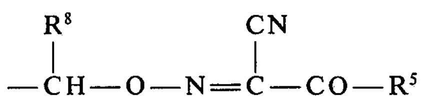

bedeutet, wobei $R^5$ die obige Bedeutung hat und $R^8$ für Wasserstoff, Methyl oder Ethyl steht.

Von den erfindungsgemäßen substituierten Oximinoacetaniliden der Formel (I) sind besonders bevorzugt diejenigen, bei denen

$R^1$ für Alkyl mit 1 bis 4 C-Atomen, insbesondere Methyl und Ethyl, für Alkoxy mit 1 bis 4 Kohlenstoffatomen, wie Methoxy oder Ethoxy, oder Chlor steht,
$R^2$ für Wasserstoff, Alkyl mit 1 bis 4 C-Atomen, insbesondere Methyl oder Ethyl, oder Chlor steht,
$R^3$ und $R^4$ für Wasserstoff stehen,
$R^5$ für Alkoxy mit 1 bis 3 C-Atomen oder für Amino steht und
$R^6$ $C_3$—$C_5$-Alkinyl, $C_1$—$C_4$-Alkoxy-$C_1$—$C_3$-alkyl, $C_1$—$C_4$-Alkylthio-$C_1$—$C_3$-alkyl, Halogen-$C_1$—$C_4$-alkoxy-$C_1$—$C_3$-alkyl, Cyan-$C_1$—$C_3$-alkoxy-$C_1$—$C_3$-alkyl, $C_4$—$C_7$-Cycloalkoxy-$C_1$—$C_3$-alkyl, $C_2$—$C_4$-Alkenoxy-$C_1$—$C_3$-alkyl, $C_2$—$C_4$-Alkinoxy-$C_1$—$C_3$-alkyl, Methylcarbonylmethyl, Methoxycarbonyl-$C_1$—$C_3$-alkyl, Methylthiocarbonyl-$C_1$—$C_3$-alkyl bedeuten, außerdem Aminocarbonyl-$C_1$—$C_3$-alkyl, wobei die Aminogruppe durch 1—2 Methylreste substituiert oder Bestandteil eines 5- oder 6-gliedrigen heterocyclischen Rings sein kann, ferner ein 5- oder 6-gliedriger über C- oder N-verknüpfter Heterocyclyl-$C_1$—$C_4$-alkyl-Rest, der bis zu 3 Stickstoffatomen, bis zu 2 Sauerstoffatomen oder bis zu 2 Stickstoff- und 1 Sauerstoffatom enthalten kann, einen Bicycloheterocyclyl-$C_1$—$C_4$-alkylrest mit bis zu 3 Stickstoffatomen, wobei die heterocyclischen Reste durch Methyl-, Ethyl- oder die Ketogruppe weiter substituiert sein können, ein hydrierter Furanonrest oder ein Methoxycarbonyl-(cyan)-methyleniminoxymethyl- bzw. Aminocarbonyl-(cyan)-methyleniminoxymethyl-Rest bedeutet.

Die angeführten Reste können ein- oder mehrfach, gleich oder verschieden substituiert sein.

Neben den in den Herstellungsbeispielen genannten erfindungsgemäßen Verbindungen seien beispielhaft folgende weitere erfindungsgemäßen Verbindungen der allgemeinen Formel (I) genannt:

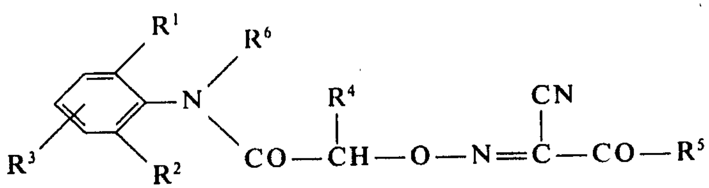
(I)

| $R^1$ | $R^2$ | $R^3$ | $R^4$ | $R^5$ | $R^6$ |
|---|---|---|---|---|---|
| $CH_3$ | $CH_3$ | H | H | $OC_4H_9$-n | $CH_2—OC_2H_5$ |
| $CH_3$ | $CH_3$ | H | $CH_3$ | $OC_4H_9$-n | $CH_2—OC_2H_5$ |
| $CH_3$ | $CH_3$ | H | H | $NH_2$ | $CH_2—O—CH_2—CH(CH_3)_2$ |
| $CH_3$ | $CH_3$ | H | H | $NH—CO—NH_2$ | $CH_2—O—CH_2—CH(CH_3)_2$ |
| $CH_3$ | $CH_3$ | H | H | $NH_2$ | 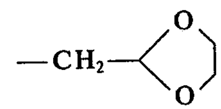 |
| $CH_3$ | $CH_3$ | H | H | $NH—CO—NH—C_2H_5$ | 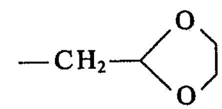 |
| $CH_3$ | $CH_3$ | $CH_3$ | H | $NH_2$ | $CH_2—OCH_3$ |
| $CH_3$ | $CH_3$ | H | H | $NH_2$ | $CH(C_2H_5)—CO—OCH_3$ |
| $CH_3$ | $CH_3$ | H | H | $NH_2$ | $CH(CH_3)—O—N{=}C(CN)—CO—OC_2H_5$ |
| $CH_3$ | $CH_3$ | H | H | $NH—CO—NH_2$ | $CH(C_2H_5)—O—N{=}C—(CN)—CO—OCH_3$ |
| $CH_3$ | $C_2H_5$ | H | H | $NH—CO—NH_2$ | $CH_2—C{\equiv}CH$ |
| $CH_3$ | $C_2H_5$ | H | H | $NH—CO—NH(CH_2)_5—CN$ | $CH_2—OC_2H_5$ |
| $CH_3$ | $C_2H_5$ | H | $CH_3$ | $OCH_3$ | $CH_2—OC_2H_5$ |
| $CH_3$ | $C_2H_5$ | H | H | $NH_2$ | $CH(CH_3)—CH_2—OCH_3$ |

Fortsetzung

| $R^1$ | $R^2$ | $R^3$ | $R^4$ | $R^5$ | $R^6$ |
|---|---|---|---|---|---|
| $CH_3$ | $C_2H_5$ | H | H | $OC_2H_5$ | $CH(CH_3)—CH_2—OCH_3$ |
| $CH_3$ | $C_2H_5$ | H | H | $NH—CO—NH—CH_3$ | $CH_2—CO—OCH(CH_3)_2$ |
| $C_2H_5$ | $C_2H_5$ | H | H | $NH—CO—NH—C_6H_{11}$ | $CH_2—OCH_3$ |
| $C_2H_5$ | $C_2H_5$ | H | H | $NH—CO—NH—(CH_2)_2—OCH_3$ | $CH_2—OCH_3$ |
| $C_2H_5$ | $C_2H_5$ | H | H | $NH—CO—NH—(CH_2)_2—CO—OCH_3$ | $CH_2—OC_4H_9$-n |
| $C_2H_5$ | $C_2H_5$ | H | H | $NH—CO—NH—CH_2—$ 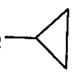 | $CH_2—OC_4H_9$-n |
| $C_2H_5$ | $C_2H_5$ | H | H | $NH—CO—NH_2$ | $CH_2—CH_2—OC_3H_7$-n |
| $C_2H_5$ | $C_2H_5$ | H | H | $OC_2H_5$ | $CH_2—CH_2—OC_3H_7$-n |
| $C_2H_5$ | $C_2H_5$ | H | H | $NH_2$ | $CH_2—CO—OC_2H_5$ |
| $C_2H_5$ | $C_2H_5$ | H | H | $NH—CO—NH—(CH_2)_2—OCH_3$ | $CH_2—CO—OC_2H_5$ |
| $CH_3$ | $C_4H_9$-t | H | H | $OCH_3$ | $CH_2—OC_4H_9$-n |
| $CH_3$ | $C_4H_9$-t | H | $CH_3$ | $NH_2$ | $CH_2—OC_4H_9$-n |
| $CH_3$ | $CH_3$ | H | H | $OC_2H_5$ | $—CH_2—$ 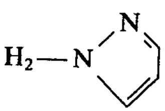 |
| $CH_3$ | $CH_3$ | H | H | $NH—CO—NH—C_2H_5$ | $—CH_2—$ 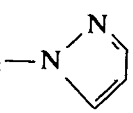 |
| $CH_3$ | $CH_3$ | $CH_3$ | H | $NH_2$ | $—CH_2—$ 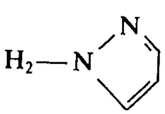 |

Fortsetzung

| $R^1$ | $R^2$ | $R^3$ | $R^4$ | $R^5$ | $R^6$ |
|---|---|---|---|---|---|
| $CH_3$ | $CH_3$ | $CH_3$ | H | $OCH_3$ | 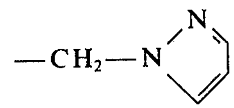 |
| $CH_3$ | $C_2H_5$ | H | H | $NH—CO—NH—CH_3$ | 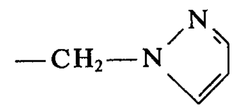 |
| $CH_3$ | $C_2H_5$ | H | H | $NH—CO—NH—C_2H_5$ | 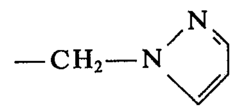 |
| $CH_3$ | $CH_3$ | H | H | $NH_2$ | 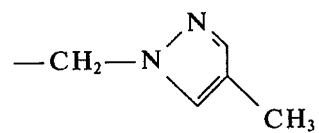 |
| $CH_3$ | $CH_3$ | H | H | $OCH_3$ | 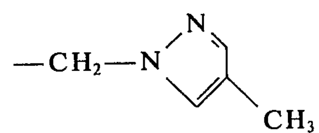 |
| $CH_3$ | $C_2H_5$ | H | H | $NH_2$ | 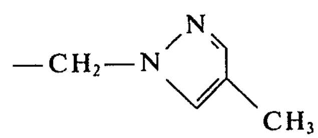 |
| $CH_3$ | $C_2H_5$ | H | H | $NH—CO—NH_2$ | 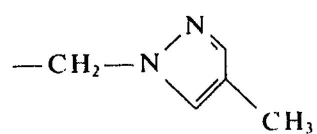 |

Fortsetzung

| $R^1$ | $R^2$ | $R^3$ | $R^4$ | $R^5$ | $R^6$ |
|---|---|---|---|---|---|
| $CH_3$ | $CH_3$ | H | H | $NH-CO-NH_2$ | 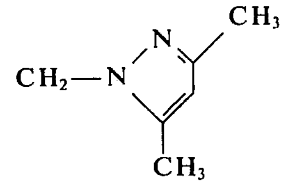 |
| $CH_3$ | $CH_3$ | H | H | $OC_2H_5$ | 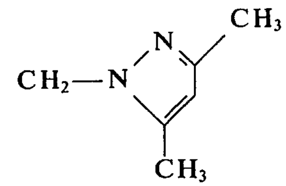 |
| $CH_3$ | $CH_3$ | H | H | $NH-CO-NH_2$ | 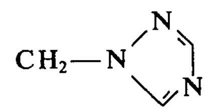 |
| $CH_3$ | $CH_3$ | H | H | $NH-CO-NH-C_6H_{11}$ | 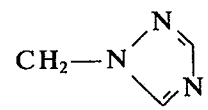 |
| $CH_3$ | $CH_3$ | H | H | $OC_4H_9$-n | 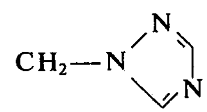 |
| $CH_3$ | $CH_3$ | H | H | $NH-CO-NH_2$ | $CH_2-O-N{=}C(CN)CO-NH_2$ |
| $CH_3$ | $CH_3$ | H | H | $NH-CO-NH-CH_3$ | $CH_2-O-N{=}C(CN)CO-OCH_3$ |
| $C_2H_5$ | $C_2H_5$ | H | H | $OCH_3$ | $CH_2-O-N{=}C(CN)CO-NH_2$ |
| $C_2H_5$ | $C_2H_5$ | H | H | $OC_2H_5$ | $CH_2-O-N{=}C(CN)CO-NH_2$ |

Verwendet man nach Verfahrensvariante a) beispielsweise N-n-Butylthiomethyl-N-(2,6-dimethylphenyl)-2-chloracetamid und 2-Cyan-2-oximinoessigsäureethylester als Ausgangsstoffe und N-Ethyl-N,N-diisopropylamin als Protonenakzeptor, so kann der Reaktionsverlauf durch das folgende Formelschema wiedergegeben werden:

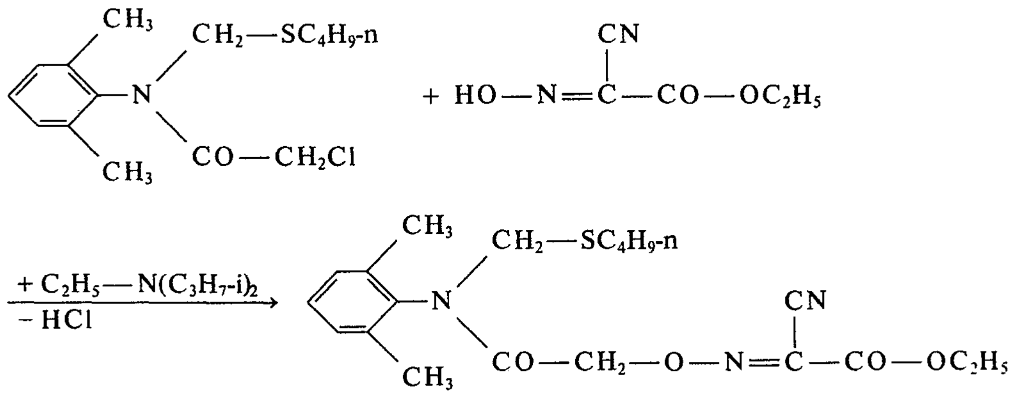


Verwendet man nach der Verfahrensvariante b) beispielsweise 2-Cyan-2-[N-(n-butylthiomethyl)-N-(2,6-dimethylphenyl)-aminocarbonylmethoximino]-acetamid und n-Propylisocyanat als Ausgangsstoffe und Natriumhydrid als Base, so kann der Reaktionsverlauf gemäß folgendem Schema formuliert werden:

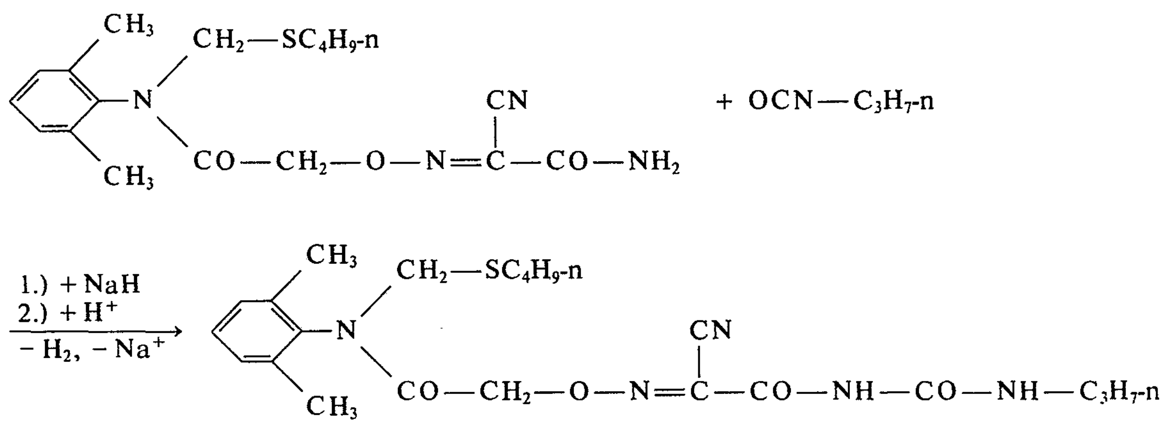


Verwendet man nach der Verfahrensvariante c) beispielsweise N-Chlormethyl-N-chloracetyl-2,6-dimethyl-anilin und 2-Cyan-2-oximinoessigsäuremethylester als Ausgangsstoffe und Triethylamin als Protonakzeptor, so kann der Reaktionsverlauf gemäß folgendem Schema formuliert werden:

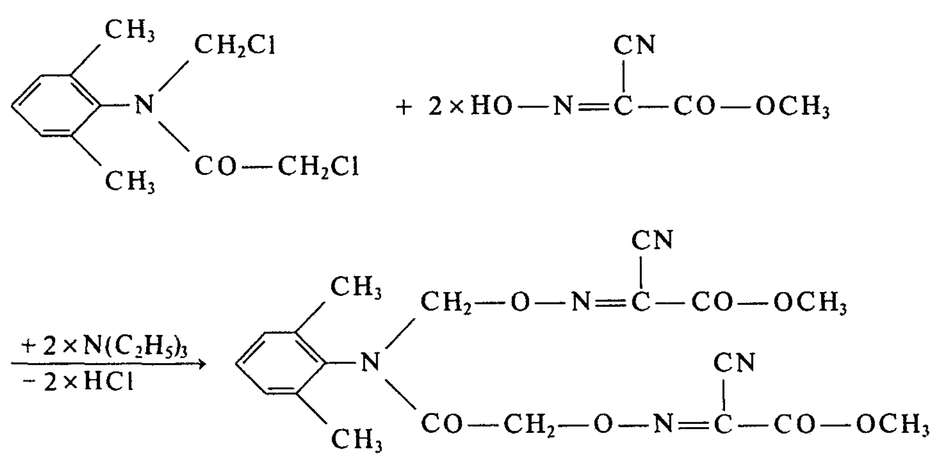


Die bei der Durchführung der Verfahrensvariante a) als Ausgangsstoffe eingesetzten substituierten Acetanilide sind durch Formel (II) allgemein definiert. In dieser Formel haben die Reste $R^1$ bis $R^4$, $R^6$ und X die Bedeutung, die bereits im Zusammenhang mit Formel (I) genannt wurde.

Die substituierten Acetanilide der Formel (II) sind bekannt oder können nach prinzipiell bekannten Verfahren, beispielsweise durch Umsetzung von N-substituierten Anilinen mit Säurehalogeniden oder aber durch Reaktion von sekundären Acetaniliden mit Verbindungen der Struktur

$X-R^6$

wobei
X und $R^6$ die obigen Bedeutungen haben,
gegebenenfalls unter Phasentransferbedingungen erhalten werden (vgl. z. B. Europa-Patent 29 011, US-PS 3 997 326, DE-PS 2 328 340, DE-OS 2 405 510).

Einige Verbindungen, die unter die Struktur

$X-R^6$

fallen, sind neu ebenso wie die entsprechenden Vorprodukte, es handelt sich um die Verbindung der Formel

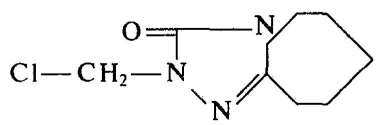

die aus der ebenfalls neuen Hydroxymethyl-Verbindung durch Umsetzung mit Thionylchlorid hergestellt werden kann.

Die u. a. als Ausgangsprodukte bei der Verfahrensvariante a) benötigten Verbindungen der Formel (IIa)

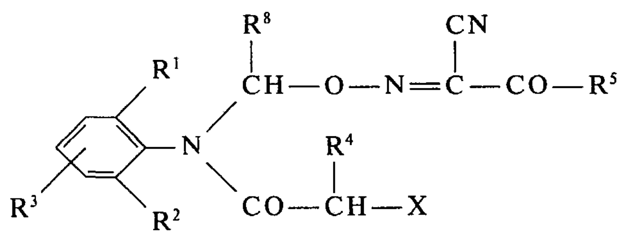

(IIa)

in der
$R^1$, $R^2$, $R^3$, $R^4$, $R^5$, $R^8$ und X die obigen Bedeutungen haben,
können z. B. durch Umsetzung von einer Verbindung der Formel (VI)

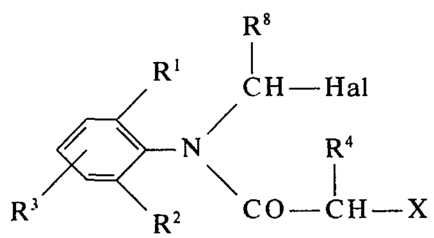

(VI)

in der
$R^1$, $R^2$, $R^3$, $R^4$, $R^8$ Hal und X die obigen Bedeutungen haben,
mit einem 2-Cyan-2-oximinoessigsäurederivat der Formel (III) und einem Protonenakzeptor, wie z. B. Triethylamin, in einem indifferenten Lösungsmittel, wie Acetonitril oder Toluol, bei niedriger Temperatur, z. B. bei $-20$ bis 0°C erhalten werden.

Die weiterhin bei der Durchführung der Verfahrensvarianten a) und b) zu verwendenden 2-Cyan-2-oximino-essigsäurederivate sind durch die Formel (III) allgemein definiert. In dieser Formel hat der Rest $R^5$ die unter Formel (I) angegebene Bedeutung. Diese Verbindungen sind größtenteils bekannt oder können nach bekannten Verfahren hergestellt werden (vgl. Ber. 42, 736–741 (1909); 54, 1334 (1921); US-PS 4 188 401).

So erhält man beispielsweise oximierte Harnstoffe der Formel (IIIa)

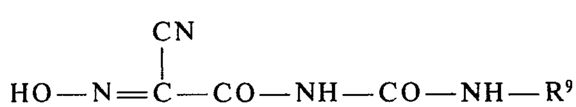

(IIIa)

in welcher
$R^9$ die oben angegebene Bedeutung hat,

wenn man Isocyanate der Formel (V)

$$R^9—NCO \quad (V)$$

in welcher
$R^9$ die oben angegebene Bedeutung hat,
in einer ersten Stufe mit Ammoniak zu N-substituiertem Harnstoff umsetzt, in einer zweiten Stufe diesen mit Cyanessigsäure in Gegenwart von Acetanhydrid zum entsprechenden 1-substituierten 3-(2-Cyanacetyl)-harnstoff und diesen mit salpetriger Säure zum entsprechenden oximierten Harnstoff weiter umsetzt.

Die bei der Durchführung der Verfahrensvariante b) benötigten Verbindungen der Formel (IV) können nach der oben beschriebenen Methode aus II und III hergestellt werden.

Die ebenfalls benötigten Isocyanate der Formel (V) bei der Verfahrensvariante b) sind bekannte Verbindungen und können in üblicher Weise, z. B. durch Umsetzung von primären Aminen mit Phosgen hergestellt werden.

Die bei der Durchführung der Verfahrensvariante c) einzusetzenden Verbindungen der Formel (VI) können wie bereits weiter oben beschrieben nach Analogieverfahren aus Anilinen mit Aldehyden zu den Schiffschen Basen umgesetzt werden, die dann mit einem Säurehalogenid weiter umgesetzt werden zu den Verbindungen der Formel (VI) (vgl. z. B. Europa-Patent 29 011).

Als Verdünnungsmittel können bei der Verfahrensvariante a) alle gegenüber den Reaktionspartnern inerten organischen Lösungsmittel in Frage kommen, vorzugsweise polare Lösungsmittel, beispielsweise Acetonitril, Aceton, Chloroform, Benzonitril, Dimethylacetamid, Dimethylformamid, Dimethylsulfoxid, Chlorbenzol, Essigsäureethylester, Dioxan, Methylethylketon, Methylenchlorid und Tetrahydrofuran und unpolare Lösungsmittel, beispielsweise Toluol.

Die Umsetzung kann gegebenenfalls auch in Mischungen aus Wasser und einem wassermischbaren organischen Lösungsmittel durchgeführt werden oder in heterogenen Systemen, bestehend aus Wasser und einem mit Wasser nicht mischbaren oder nur teilweise mischbaren Lösungsmittel.

Als Säurebinder werden organische Basen, vorzugsweise tertiäre Amine, beispielsweise Chinolin, Dimethylbenzylamin, N,N-Dimethylanilin, Ethyldicyclohexylamin, Ethyldiisopropylamin, Picolin, Pyridin und Triethylamin verwendet oder die 2-Cyan-2-oximinoessigsäurederivate werden in Form ihrer Alkali- oder Erdalkalisalze eingesetzt.

Die erfindungsgemäße Umsetzung wird bei einer Temperatur von 0 bis 140°C, bevorzugt von 60 bis 110°C ausgeführt. Bei Verwendung oder Mitverwendung von Wasser als Verdünnungsmittel wird bei einer Temperatur vom Erstarrungspunkt der wäßrigen Lösung um etwa +70°C, vorzugsweise von 40 bis 60°C umgesetzt.

Das erfindungsgemäße Verfahren kann beispielsweise wie folgt ausgeführt werden:
Ein 2-Cyan-2-oximinoessigsäure-Derivat der Formel (III) wird in einem Verdünnungsmittel dispergiert bzw. gelöst vorgelegt und mit einer molaren Menge eines tertiären Amins versetzt, wobei Salzbildung eintreten kann. Wird ein Alkali- oder Erdalkalisalz des 2-Cyan-2-oximinoessigsäure-Derivats eingesetzt, kann dieses in einem inerten Lösungsmittel vorgelegt werden. Dazu wird das substituierte Acetanilid der Formel (II), vorzugsweise gelöst in einem Verdünnungsmittel, gegeben. Gegebenenfalls gibt man zum Umsetzungsgemisch zur schnelleren Reaktion eine kleine Menge eines lodids. Nach Beendigung der Umsetzung werden die substituierten Oximinoacetanilide auf übliche Art isoliert und falls erforderlich gereinigt.

Als Verdünnungsmittel bei der Verfahrensvariante b) können indifferente wasserfreie Lösungsmittel wie Ether, beispielsweise Diisopropylether, Dioxan oder Tetrahydrofuran verwendet werden.

Die Umsetzung wird bei einer Temperatur von —20 bis 80°C, vorzugsweise 20 bis 60°C, durchgeführt.

Als Basen können Alkoholate, beispielsweise Alkalialkoholate wie Natriummethoxid und Kalium-tert.-butoxid und Metallhydride, beispielsweise Alkalihydride wie Natriumhydrid und Kaliumhydrid eingesetzt werden.

Die erfindungsgemäße Verfahrensvariante b) wird wie folgt ausgeführt:
Eine Verbindung der Formel (IV) wird in einem Verdünnungsmittel mit einer der vorstehend genannten Basen zum entsprechenden Anion des Amids umgesetzt und dieses mit dem Isocyanat der Formel (V) in dem angegebenen Temperaturbereich umgesetzt. Nach beendeter Reaktion wird in der Kälte mit einer organischen Carbonsäure wie Essigsäure schwach angesäuert.

Verfahrensvariante c) kann ebenfalls in einem der oben angeführten inerten Lösungsmittel in Gegenwart eines Säurebindemittels wie unter a) und b) beschrieben bei erhöhter Temperatur umgesetzt werden. Man geht gewöhnlich so vor, daß man eine Verbindung der Formel (VI) mit mindestens 2 Mol eines 2-Cyan-2-oximinoessigsäure-Derivats der Formel (III) in Gegenwart eines Protonenakzeptors, wie z. B. Methyl-diisopropylamin, in einem inerten Lösungsmittel, beispielsweise Acetonitril, bei erhöhter Temperatur umsetzt.

Je nach Umsetzungsbedingungen fallen die erfindungsgemäßen Wirkstoffe kristallin aus oder sie bleiben im organischen Lösungsmittel gelöst und können dann nach Auswaschen der Lösung mit Wasser durch vorsichtiges Einengen der Lösung oder durch Zugabe wenig polarer organischer

Lösungsmittel, wie Cyclohexan, Dibutylether, Butylacetat oder Tetrachlorkohlenstoff, abgeschieden werden. Gegebenenfalls müssen mit Wasser mischbare Lösungsmittel nach der Reaktion durch Abdampfen im Vakuum entfernt werden.

Sind die erfindungsgemäßen Verbindungen in einem mit Wasser mischbaren Lösungsmittel gelöst, so können sie beispielsweise auch durch Zugabe von Wasser ausgefällt werden. Soweit es die besonderen Bedingungen der Aufarbeitungsprozesse erlauben, werden die Lösungen der erfindungsgemäßen Wirkstoffe, bzw. die noch Lösungsmittel-feuchten Suspensionen der Wirkstoffe schwach sauer eingestellt.

Die erfindungsgemäßen Verbindungen können sich bei höherer Temperatur zersetzen, so daß Schmelzpunkte nicht immer exakt ermittelt werden können.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z.B. gegen den Erreger der Kraut- und Braunfäule der Kartoffel und Tomate (Phytophthora infestans), eingesetzt werden.

Sie zeigen eine hohe kurative und protektive Wirksamkeit. Daneben sind gute Wirkungen gegen Bakterien festzustellen.

Die erfindungsgemäßen Wirkstoffe weisen nicht nur die guten Eigenschaften hervorragender Handelspräparate auf, sondern besitzen darüber hinaus noch erhebliche Vorteile. Diese liegen in erster Linie in der Fähigkeit der erfindungsgemäßen Stoffe, in die Pflanze einzudringen. Sie können aufgenommen werden von der Saatgutoberfläche, von den Wurzeln und auch von oberirdischen Pflanzenorganen nach äußerlichen Applikationen. Auch besitzen sie die vorteilhafte Fähigkeit, locosystemisch zur Wirkung zu kommen, d.h. eine Tiefenwirkung im Pflanzengewebe auszuüben und dabei pilzliche Krankheitserreger zu eliminieren, die bereits in das Gewebe der Wirtspflanze eingedrungen sind.

Die Wirkstoffe können in die üblichen Formulierungen übergeführt werden, wie Lösungen, Emulsionen, Suspensionen, Pulver, Schäume, Pasten, Granulate, Aerosole, Wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Die Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid, als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, woe hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Zusätzlich zu den obigen Formulierungsmöglichkeiten ist zu bemerken, daß die erfindungsgemäßen Stoffe zusammen mit Saccharose, Dextrose, Dextrinen, mit wasserfreiem Calciumsulfat oder Calciumsulfat-hemihydrat, sowie mit Carbonsäuren, wie z.B. Fumarsäure oder 4-Hydroxylbenzoesäure, oder auch mit schwach sauren Ionenaustauschern formuliert werden können.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90%.

Die erfindungsgemäßen Wirkstoffe können in den Formulierungen oder in den verschiedenen Anwendungsformen in Mischungen mit anderen bekannten Wirkstoffen vorliegen, wie Fungiziden, Bakteriziden, Insektiziden, Akariziden, Nematiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02% am Wirkungsort erforderlich.

## Herstellungsbeispiele

### Beispiel 1

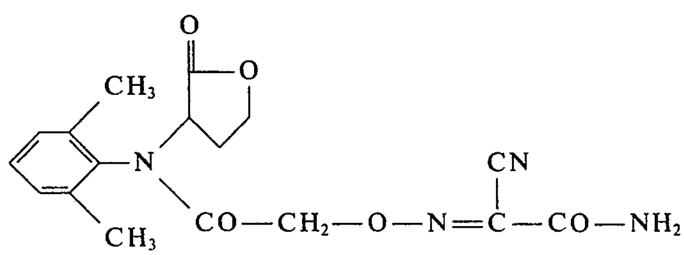

7,91 g 2-Cyan-2-oximinoacetamid und 200 g Dimethylsulfoxid werden vorgelegt und mit 7,1 g Triethylamin versetzt. Nach 30 Minuten werden 20,6 g N-(Tetrahydrofuran-2-on-3-yl)-N-(2,6-dimethylphenyl)-2-chloracetamid zugefügt und die Mischung 7 Stunden auf 115°C gehalten. Kontrolle der Umsetzung durch Dünnschichtchromatogramm auf Kieselgel 60 F 254, Fließmittel: Essigsäure-Ethylacet-Toluol. Der größte Teil des Dimethylsulfoxids wird bei 0,2 mbar abdestilliert. Der Destillationsrückstand wird mit Ethylacetat und Wasser versetzt. Die Ethylacetatlösung wird 3mal mit Wasser gewaschen, über Natriumsulfat getrocknet und im Vakuum eingedampft. Der Destillationsrückstand kristallisiert mit Diisopropylether. Man erhält 20,8 g an 2-Cyan-2-(N-(2,6-dimethylphenyl)-N-(tetrahydrofuran-2-on-3-yl)-amino-carbonylmethoximino)-acetamid mit dem Schmelzpunkt von 201–203°C. Aus Toluol-Diethylketon (7 : 1) umkristallisiert, schmilzt die Verbindung bei 203–204°C.

Analog werden die folgenden Verbindungen der Formel (I) hergestellt:

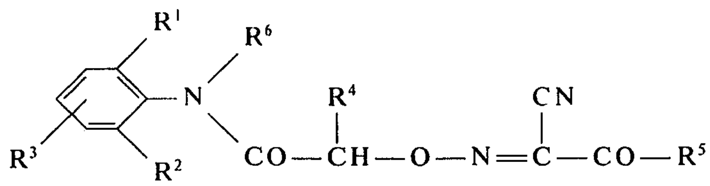

| Bsp. Nr. | $R^1$ | $R^2$ | $R^3$ | $R^4$ | $R^5$ | $R^6$ | Schmelzpunkt, °C | Reaktionsbedingungen |
|---|---|---|---|---|---|---|---|---|
| 2 | $CH_3$ | $CH_3$ | H | H | $NH_2$ | $-CH_2-OCH_3$ | 151,5 | 6 h 80° Acetonitril |
| 3 | $C_2H_5$ | $C_2H_5$ | H | H | $NH_2$ | $-CH_2-OCH_3$ | 109 | 7 h 80° Acetonitril |
| 4 | $C_2H_5$ | $C_2H_5$ | H | H | $NH_2$ | $-CH_2-O-N{=}C(CN)-CO-NH_2$ | 161 | 7 h 80° Acetonitril |
| 5 | $CH_3$ | $CH_3$ | H | H | $NH_2$ | $-CH_2-O-N{=}C(CN)-CO-NH_2$ | 228 | 7 h 80° Acetonitril |
| 6 | $CH_3$ | $CH_3$ | H | H | $NH_2$ | 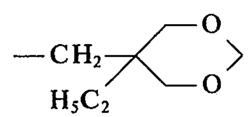 | 167 | 7 h 80° Acetonitril |
| 7 | $CH_3$ | $CH_3$ | H | H | $NH_2$ | 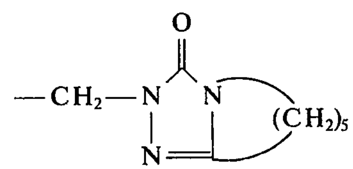 | 95 | 7 h 80° Acetonitril |
| 8 | $CH_3$ | $CH_3$ | H | $CH_3$ | $NH_2$ | $-CH_2-OCH_3$ | 137 | 5 h 117° DMSO |
| 9 | $CH_3$ | $CH_3$ | H | H | $NH_2$ | $-CH(CH_3)-CO-OCH_3$ | 171,5 | 5 h 116° DMSO |
| 10 | $C_2H_5$ | $C_2H_5$ | H | H | $NH_2$ | $-CH(CH_3)-CO-OCH_3$ | 102 | 5 h 110° DMSO |
| 11 | $CH_3$ | $CH_3$ | H | H | $NH_2$ | 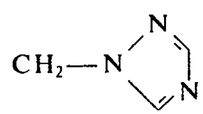 | 201,5 | 6 h 80° acetonitrile |

Fortsetzung

| Bsp. Nr. | $R^1$ | $R^2$ | $R^3$ | $R^4$ | $R^5$ | $R^6$ | Schmelzpunkt, °C | Reaktionsbedingungen |
|---|---|---|---|---|---|---|---|---|
| 12 | $CH_3$ | $CH_3$ | H | H | $NH_2$ | 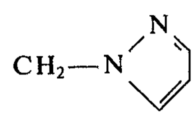 | 176 | 6 h 80° acetonitrile |
| 13 | $CH_3$ | $CH_3$ | H | H | $NH_2$ | $-CH(CH_3)-CO-OCH(CH_3)_2$ | 92 | 4 h 115° DMF |

a) Herstellung des Zwischenproduktes zu Beispiel 6

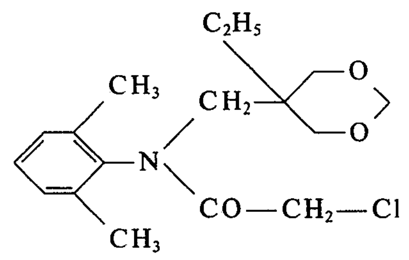

19,9 g N-((5-Ethyl-1,3-dioxan-5-yl)-methyl)-2,6-dimethylanilin, gelöst in 50 ml Chlorbenzol, werden bei 5 °C mit 6,4 g Chloracetylchlorid versetzt. Die Mischung wird 2 Stunden auf 80°C gehalten und jeweils nach 1 Stunde nochmals 3,2 g Chloracetylchlorid zugesetzt. Schließlich beläßt man die Mischung noch 5 Stunden bei 100°C. Man dampft im Vakuum ein, löst den Rückstand in Xylol und wäscht zweimal mit Wasser unter Zusatz von etwas Essigsäure aus. Die Xylollösung wird über Natriumsulfat getrocknet, zweimal mit Tonsil optimum gerührt und im Vakuum eingedampft. Der Rückstand – 22,6 g – kristallisiert langsam; er wird mit Petrolether behandelt. Die Kristalle werden bei 60°C im Vakuum getrocknet. Man erhält Kristalle mit einem Schmelzpunkt von 99°C und das IR-Spektrum ($CHCl_3$) zeigt bei 1680 $cm^{-1}$ eine N–CO-Bande.

a1) Darstellung des Vorproduktes

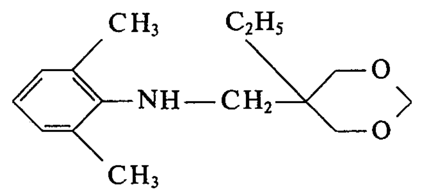

Eine Mischung aus 82,3 g 5-Ethyl-1,3-dioxan-5-yl-methyl-toluolsulfonat, 106 g Dimethylformamid, 121 g 2,6-Dimethylanilin und 40 g Kaliumcarbonat wird 16 Stunden auf 163–169°C erhitzt. Die Reaktionsmischung wird anschließend mit Toluol verdünnt, mit Wasser gewaschen, im Vakuum eingedampft und der Rückstand destilliert. Die Fraktion bei 126°C/0,04 mbar enthält 11,6 g N-((5-Ethyl-1,3-dioxan-5-yl)-methyl)-2,6-dimethylanilin.

b) Herstellung des Zwischenproduktes zu Beispiel 7

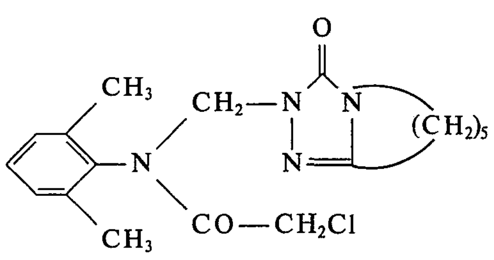

Eine Mischung aus 19,8 g N-Chloracetyl-2,6-dimethylanilin, 200 ml Methylenchlorid, 100 ml 45%iger Natronlauge, 0,5 g Tetrabutylammoniumchlorid und 22,5 g 2-Chlormethyl-3-oxo-4,5-pentamethylen-1,2,4-triazolin-5 wird bei 40°C 4½ Std. rasch gerührt. Es wird mit 600 ml Toluol versetzt, mehrfach mit Wasser gewaschen, über Natriumsulfat getrocknet und anschließend mit Tonsil optimum behandelt. Es wird im Vakuum eingedampft. Der Abdampfrückstand wird in 600 ml heißem Xylol gelöst und mit einer Mischung aus 100 ml Dibutylether und 200 ml Petrolether versetzt.

N-(2,6-Dimethylphenyl)-N-(3-oxo-4,5-pentamethylen-1,2,4-triazol-(5)-in-2-yl-methyl)-chloracetamid kristallisiert aus. Es wird abgetrennt, mit Petrolether gewaschen und getrocknet. Die Verbindung hat einen Schmelzpunkt von 128°C.

b1) Herstellung des Vorproduktes

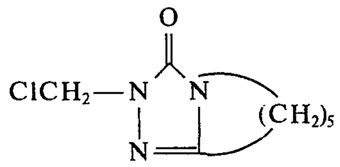

Eine Lösung von 21,7 g 2-Hydroxymethyl-3-oxo-4,5-pentamethylen-1,2,4-triazolin(5) in 50 ml Chloroform sowie ein Tropfen Dimethylformamid wird bei 3°C mit 29 g Thionylchlorid versetzt. Man läßt 4 Stunden ohne Kühlung stehen und dampft im Vakuum ein. Der feste Rückstand wird bei 30°C/0,1 mbar getrocknet. Man erhält 23,3 g 2-Chlormethyl-3-oxo-4,5-pentamethylentriazolin(5).

b2) Herstellung des Ausgangsproduktes

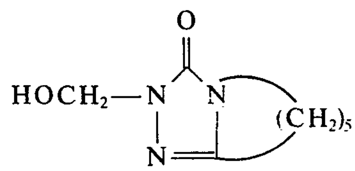

199 g 4,5-Pentamethylen-1,2,4-triazol(5)in-3-on werden in 5 Portionen bei 24°C in 290 g 24,2%ige Formalin-Lösung eingetragen. Es wird $3^1/_2$ Stunden gerührt, filtriert, mit 120 ml Wasser verdünnt und 3 × mit je 300 ml Chloroform extrahiert. Die Chloroform-Lösung wird über Natriumsulfat getrocknet, im Vakuum eingedampft und der Rückstand bei 60°C/0,1 mbar getrocknet. Man erhält 204,8 g 2-Hydroxymethyl-3-oxo-4,5-pentamethylen-1,2,4-triazolin(5) mit einem Schmelzpunkt von 104°C.

In den nachfolgenden Beispielen wird die nachstehend angegebene Verbindung als Vergleichssubstanz eingesetzt:

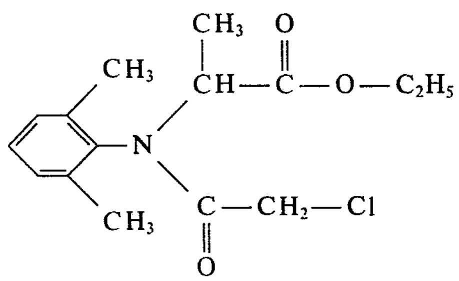

(A)

Beispiel A

Phytophthora-Test (Tomate)/protektiv

Lösungsmittel: 4,7 Gew.-Teile Aceton
Emulgator: 0,3 Gew.-Teile Alkyl-aryl-polyglykolether

Zur Herstellung einer zweckmäßigen Wirkstoffzubereitung vermischt man 1 Gew.-Teil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Bei diesem Test zeigten die erfindungsgemäßen Verbindungen eine deutliche Überlegenheit gegenüber den Verbindungen des Standes der Technik.

**Patentansprüche**

1. Substituierte Oximinoacetanilide der Formel (I)

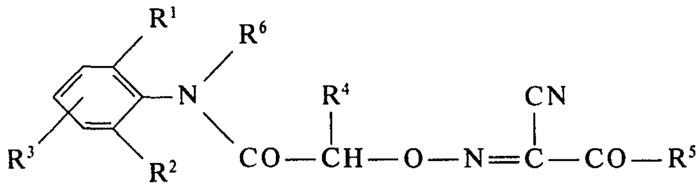

(I)

in welcher

$R^1$ Alkyl, Alkoxy oder Halogen,
$R^2$ Wasserstoff, Alkyl, Halogen oder Trifluormethyl,
$R^3$ Wasserstoff, Alkyl oder Halogen,
$R^4$ Wasserstoff oder Methyl,
$R^5$ Alkoxy, Amino oder $-NH-CO-NH-R^7$ bedeutet, wobei $R^7$ für Wasserstoff oder gegebenenfalls durch Cyan-, Alkoxy- oder Alkoxycarbonyl-substituiertes Alkyl bzw. für Cycloalkyl steht und
$R^6$ Alkinyl, Alkoxyalkyl, Halogenalkoxyalkyl, Cyanalkoxyalkyl, Cycloalkoxyalkyl, Alkenoxyalkyl, Alkinoxyalkyl, Alkylthioalkyl, Alkylcarbonylalkyl, Alkoxycarbonylalkyl, Alkylthiocarbonylalkyl bedeutet, außerdem Aminocarbonylalkyl, wobei die Aminogruppe durch Alkylreste substituiert sein kann, die auch zusammen mit dem Stickstoffatom einen heterocyclischen Ring bilden können, weiterhin ein 5- oder 6-gliedriger über C- oder N-verknüpfter Heterocyclylalkyl-Rest, der bis zu 3 Stickstoffatomen, bis zu 2 Sauerstoffatomen oder bis zu 2 Stickstoff- und 1 Sauerstoffatom enthalten kann, einen Bicycloheterocyclylalkyl-Rest mit bis zu 3 Stickstoffatomen, wobei die heterocyclischen Reste durch Alkyl- oder die Ketogruppe substituiert sein können, ferner ein hydrierter Furanon-Rest, der durch Alkylgruppen substituiert sein kann oder ein Rest der Struktur

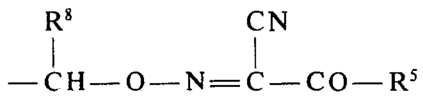

bedeutet, wobei $R^5$ die obige Bedeutung hat und $R^8$ für Wasserstoff, Methyl oder Ethyl steht.

2. Verfahren zur Herstellung von substituierten Oximinoacetaniliden der Formel (I)

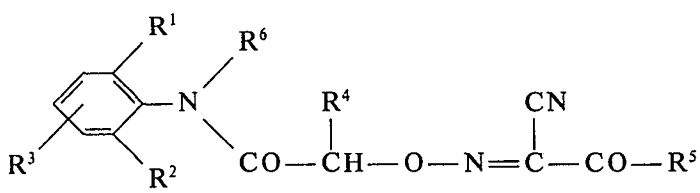

(I)

in welcher

$R^1$ Alkyl, Alkoxy oder Halogen,
$R^2$ Wasserstoff, Alkyl, Halogen oder Trifluormethyl,
$R^3$ Wasserstoff, Alkyl oder Halogen,
$R^4$ Wasserstoff oder Methyl,
$R^5$ Alkoxy, Amino oder $-NH-CO-NH-R^7$ bedeutet, wobei $R^7$ für Wasserstoff oder gegebenenfalls durch Cyan-, Alkoxy- oder Alkoxycarbonyl-substituiertes Alkyl bzw. Cycloalkyl steht und
$R^6$ Alkinyl, Alkoxyalkyl, Halogenalkoxyalkyl, Cyanalkoxyalkyl, Cycloalkoxyalkyl, Alkenoxyalkyl, Alkinoxyalkyl, Alkylthioalkyl, Alkylcarbonylalkyl, Alkoxycarbonylalkyl, Alkylthiocarbonylalkyl bedeutet, außerdem Aminocarbonylalkyl, wobei die Aminogruppe durch Alkylreste substituiert sein kann, die auch zusammen mit dem Stickstoffatom einen heterocyclischen Ring bilden können, weiterhin ein 5- oder 6-gliedriger über C- oder N-verknüpfter Heterocyclylalkyl-Rest, der bis zu 3 Stickstoffatomen, bis zu 2 Sauerstoffatomen oder bis zu 2 Stickstoff- und 1 Sauerstoffatom enthalten kann, einen Bicycloheterocyclylalkyl-Rest mit bis zu 3 Stickstoffatomen, wobei die heterocyclischen Reste durch Alkyl- oder die Ketogruppe substituiert sein können, ferner ein hydrierter Furanon-Rest, der durch Alkylgruppen substituiert sein kann oder ein Rest der Struktur

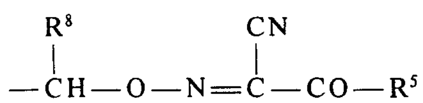

bedeutet, wobei $R^5$ die obige Bedeutung hat und $R^8$ für Wasserstoff, Methyl oder Ethyl steht,

dadurch gekennzeichnet, daß man

a) ein substituiertes Acetanilid der Formel (II)

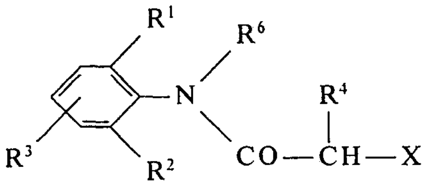

(II)

in welcher

$R^1$, $R^2$, $R^3$, $R^4$ und $R^6$ die obigen Bedeutungen haben und
X für Chlor, Brom, Jod oder für einen Sulfonyloxyrest steht,

mit einem 2-Cyan-2-oximinoessigsäurederivat der Formel (III)

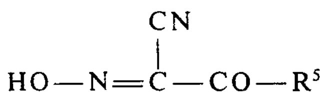

(III)

in welcher

$R^5$ die obige Bedeutung besitzt, gegebenenfalls in einem Verdünnungsmittel in Form der Alkali- oder Erdalkalisalze oder in Gegenwart eines Protonenakzeptors umsetzt, oder

b) für den Fall, daß $R^5$ für –NH–CO–NHR$^9$ steht und

$R^9$ für einen gegebenenfalls durch Cyan-, Alkoxy- oder Alkoxycarbonyl-substituierten Alkyl- oder für einen Cycloalkyl-Rest steht, aber
$R^6$ nicht für

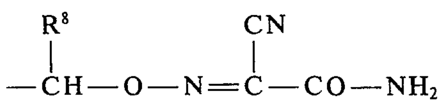

bzw.

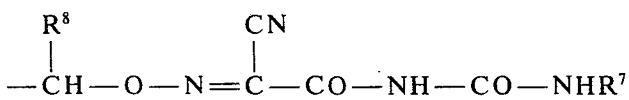

steht,

Verbindungen der Formel (IV)

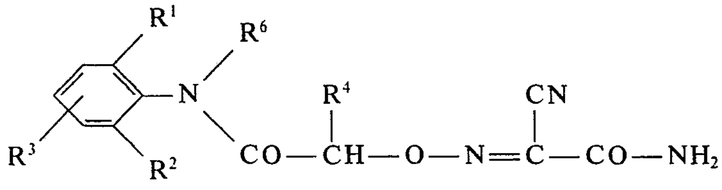

(IV)

in welcher

$R^1$, $R^2$, $R^3$ und $R^4$ die obigen Bedeutungen haben,
$R^6$ die obengenannte Bedeutung hat, aber nicht für

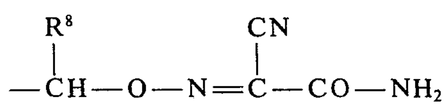

bzw.

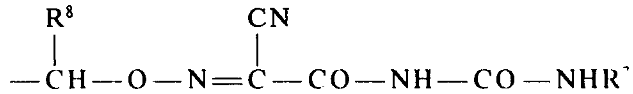

steht,

in Gegenwart einer Base mit einem Isocyanat der Formel (V)

OCN–$R^9$ (V)

in welcher

$R^9$ die obige Bedeutung hat, gegebenenfalls in Gegenwart eines Verdünnungsmittels umsetzt, oder

c) für den Fall, daß $R^6$ für

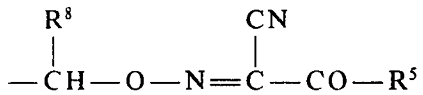

steht und $R^5$ in beiden Resten identisch ist, Verbindungen der Formel (VI)

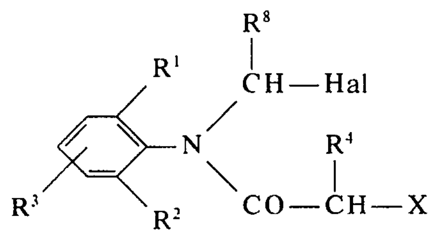

(VI)

in welcher

$R^1$, $R^2$, $R^3$, $R^4$, $R^8$ und X die obigen Bedeutungen haben und
Hal für Halogen, vorzugsweise Chlor steht,

mit 2 Mol eines 2-Cyan-2-oximinoessigsäurederivats der Formel (III) gegebenenfalls in Gegenwart eines Verdünnungsmittels in Form der Alkali- oder Erdalkalisalze oder in Gegenwart eines Protonenakzeptors umsetzt.

3. Schädlingsbekämpfungsmittel, gekennzeichnet durch einen Gehalt an mindestens einem substituierten Oximinoacetanilid der Formel (I) gemäß Ansprüche 1 und 2.

4. Verwendung von substituierten Oximinoacetaniliden der Formel (I) gemäß Ansprüche 1 und 2 zur Bekämpfung von Schädlingen.

5. Verfahren zur Bekämpfung von Schädlingen, dadurch gekennzeichnet, daß man substituierte Oximinoacetanilide der Formel (I) gemäß Ansprüche 1 und 2 auf Schädlinge und/oder ihren Lebensraum einwirken läßt.

6. Verfahren zur Herstellung von Schädlingsbekämpfungsmitteln, dadurch gekennzeichnet, daß man substituierte Oximinoacetanilide der Formel (I) gemäß Ansprüche 1 und 2 mit Streckmitteln und/oder oberflächenaktiven Mitteln vermischt.

**Claims**

1. Substituted oximinoacetanilides of the formula (I)

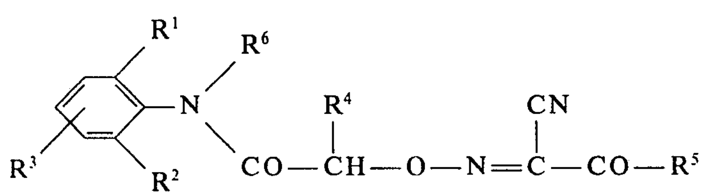

(I)

in which

$R^1$ denotes alkyl, alkoxy or halogen,
$R^2$ denotes hydrogen, alkyl, halogen or trifluoromethyl,
$R^3$ denotes hydrogen, alkyl or halogen,
$R^4$ denotes hydrogen or methyl,
$R^5$ denotes alkoxy, amino or —NH—CO—NH—$R^7$, wherein $R^7$ represents hydrogen or optionally cyano-, alkoxy- or alkoxycarbonyl-substituted alkyl, or represents cycloalkyl, and
$R^6$ denotes alkinyl, alkoxyalkyl, halogenoalkoxyalkyl, cyanoalkoxyalkyl, cycloalkoxyalkyl, alkenoxyalkyl, alkinoxyalkyl, alkylthioalkyl, alkylcarbonylalkyl, alkoxycarbonylalkyl or alkylthiocarbonylalkyl, and in addition aminocarbonylalkyl, it being possible for the amino group to be substituted by alkyl radicals which, together with the nitrogen atom, can also form a heterocyclic ring, and furthermore denotes a 5-membered or 6-membered heterocyclylalkyl radical which is bonded via C or N and which can contain up to 3 nitrogen atoms, up to 2 oxygen atoms, or up to 2 nitrogen atoms and 1 oxygen atom, or denotes a bicycloheterocyclylalkyl radical having up to 3 nitrogen atoms, it being possible for the heterocyclic radicals to be substituted by alkyl or the keto group, or furthermore denotes a hydrogenated furanone radical which can be substituted by alkyl groups, or denotes a radical of the structure

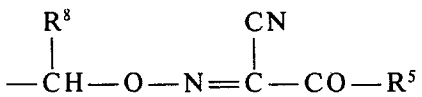

wherein $R^5$ has the above meaning and $R^8$ represents hydrogen, methyl or ethyl.

2. Process for the preparation of substituted oximinoacetanilides of the formula (I)

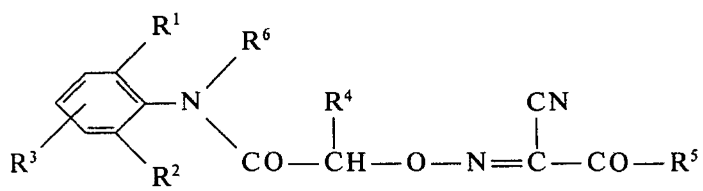

(I)

in which

$R^1$ denotes alkyl, alkoxy or halogen,
$R^2$ denotes hydrogen, alkyl, halogen or trifluoromethyl,
$R^3$ denotes hydrogen, alkyl or halogen,
$R^4$ denotes hydrogen or methyl,
$R^5$ denotes alkoxy, amino or —NH—CO—NH—$R^7$, wherein $R^7$ represents hydrogen or optionally cyano-, alkoxy- or alkoxycarbonyl-substituted alkyl, or cycloalkyl, and
$R^6$ denotes alkinyl, alkoxyalkyl, halogenoalkoxyalkyl, cyanoalkoxyalkyl, cycloalkoxyalkyl, alkenoxyalkyl, alkinoxyalkyl, alkylthioalkyl, alkylcarbonylalkyl, alkoxycarbonylalkyl or alkylthiocarbonylalkyl, and in addition aminocarbonylalkyl, it being possible for the amino group to be substituted by alkyl radicals which, together with the nitrogen atom, can also form a heterocyclic ring, and furthermore denotes a 5-membered or 6-membered heterocyclylalkyl radical which is bonded via C or N and which can contain up to 3 nitrogen atoms, up to 2 oxygen atoms, or up to 2 nitrogen atoms and 1 oxygen atom, or denotes a bicycloheterocyclylalkyl radical having up to 3 nitrogen atoms, it being possible for the heterocyclic radicals to be substituted by alkyl or the keto group, or furthermore denotes a hydrogenated furanone radical which can be substituted by alkyl groups, or denotes a radical of the structure

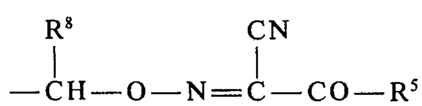

wherein $R^5$ has the above meaning and $R^8$ represents hydrogen, methyl or ethyl,

characterised in that

a) a substituted acetanilide of the formula (II)

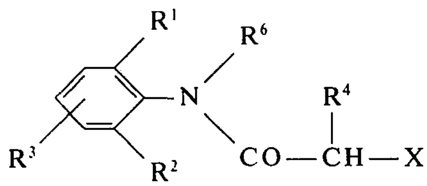

(II)

in which

$R^1$, $R^2$, $R^3$, $R^4$ and $R^6$ have the above meanings and
X represents chlorine, bromine, iodine or a sulphonyloxy radical,

is reacted with a 2-cyano-2-oximinoacetic acid derivative of the formula (III)

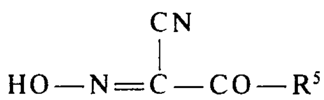

(III)

in which

$R^5$ has the above meaning, if appropriate in a diluent in the form of the alkali metal salts or alkaline earth metal salts or in the presence of a proton acceptor, or

b) in the case in which $R^5$ represents –NH–CO–NHR$^9$ and

$R^9$ represents an optionally cyano-, alkoxy- or alkoxycarbonyl-substituted alkyl radical, or represents a cycloalkyl radical, but
$R^6$ does not represent

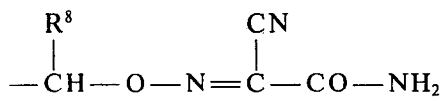

or

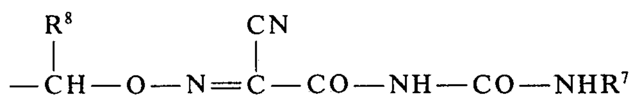

compounds of the formula (IV)

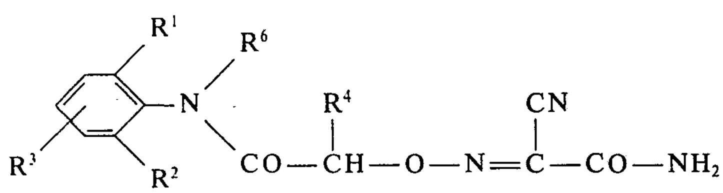

(IV)

in which

$R^1$, $R^2$, $R^3$ and $R^4$ have the above meanings, and
$R^6$ has the abovementioned meaning but does not represent

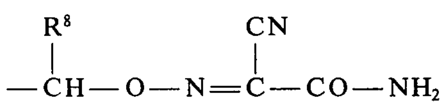

or

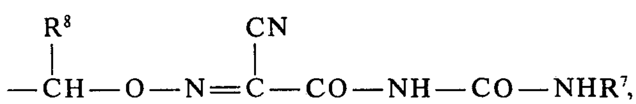

in the presence of a base, are reacted with an isocyanate of the formula (V)

OCN–$R^9$ (V)

in which

$R^9$ has the above meaning, if appropriate in the presence of a diluent, or

c) in the case in which $R^6$ represents

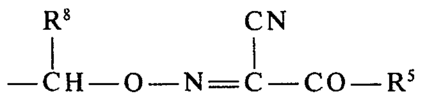

and $R^5$ is identical in both radicals, compounds of the formula (VI)

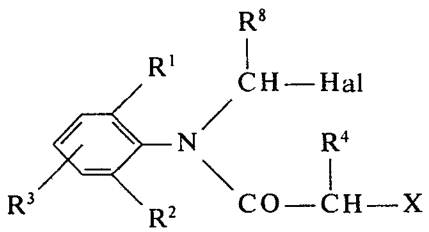

(VI)

in which

$R^1$, $R^2$, $R^3$, $R^4$, $R^8$ and X have the above meanings and
Hal represents halogen, preferably chlorine,

are reacted with 2 mols of a 2-cyano-2-oximinoacetic acid derivative of the formula (III), if appropriate in the presence of a diluent in the form of the alkali metal salts or alkaline earth metal salts or in the presence of a proton acceptor.

3. Pest-combating agents, characterised in that they contain at least one substituted oximinoacetanilide of the formula (I) according to Claims 1 and 2.

4. Use of substituted oximinoacetanilides of the formula (I) according to Claims 1 and 2 for combating pests.

5. Process for combating pests, characterised in that substituted oximinoacetanilides of the formula (I) according to Claims 1 and 2 are allowed to act on pests and/or their habitat.

6. Process for the preparation of pest-combating agents, characterised in that substituted oximinoacetanilides of the formula (I) according to Claims 1 and 2 are mixed with extenders and/or surface-active agents.

## Revendications

1. Oximino-acétanilides substitués de formule (I)

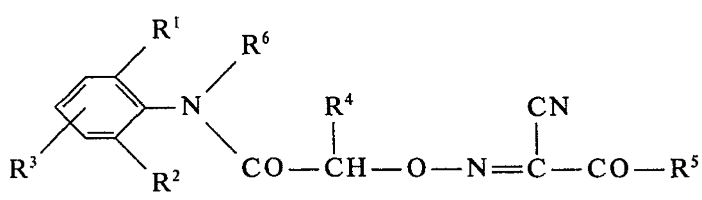

(I)

dans laquelle

$R^1$ représente un groupe alkyle, un groupe alcoxy ou un atome d'halogène,
$R^2$ représente un atome d'hydrogène, un groupe alkyle, un atome d'halogène ou un groupe trifluorométhyle,
$R^3$ représente un atome d'hydrogène, un groupe alkyle ou un atome d'halogène,
$R^4$ représente un atome d'hydrogène ou un groupe méthyle,
$R^5$ représente un groupe alcoxy, un groupe amino ou un groupe –NH–CO–NH–$R^7$, $R^7$ représentant un atome d'hydrogène ou un groupe alkyle éventuellement substitué par un groupe cyano, par un groupe alcoxy, ou par un groupe alcoxycarbonyle, ou encore un groupe cycloalkyle, et

$R^6$ représente un groupe alcynyle, un groupe alcoxyalkyle, un groupe halogénalcoxyalkyle, un groupe cyanalcoxyalkyle, un groupe cycloalcoxyalkyle, un groupe alcénoxyalkyle, un groupe alcynoxy-alkyle, un groupe alkylthioalkyle, un groupe alkylcarbonylalkyle, un groupe alcoxycarbonylalkyle, un groupe alkylthiocarbonylalkyle, de même qu'un groupe aminocarbonylalkyle, le groupe amino pouvant être substitué par des groupes alkyle qui, ensemble avec l'atome d'azote, peuvent également former un noyau hétérocyclique, ainsi qu'un groupe hétérocyclylalkyle pentagonal ou hexagonal relié par C ou N et pouvant contenir jusqu'à 3 atomes d'azote, jusqu'à 2 atomes d'oxygène ou jusqu'à 2 atomes d'azote et 1 atome d'oxygène, un groupe bicyclohétérocyclylalkyle contenant jusqu'à 3 atomes d'azote, les groupes hétérocycliques pouvant être substitués par des groupes alkyle ou par le groupe céto, de même qu'un groupe furanone hydrogéné pouvant être substitué par des groupes alkyle ou un radical de structure

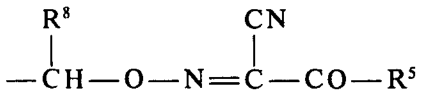

$R^5$ ayant la signification indiquée ci-dessus et $R^8$ représentant un atome d'hydrogène, un groupe méthyle ou un groupe éthyle.

2. Procédé de préparation d'oximino-acétanilides substitués de formule (I)

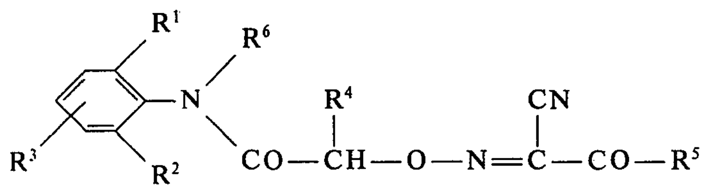

(I)

dans laquelle

$R^1$ représente un groupe alkyle, un groupe alcoxy ou un atome d'halogène,
$R^2$ représente un atome d'hydrogène, un groupe alkyle, un atome d'halogène ou un groupe trifluorométhyle,
$R^3$ représente un atome d'hydrogène, un groupe alkyle ou un atome d'halogène,
$R^4$ représente un atome d'hydrogène ou un groupe méthyle,
$R^5$ représente un groupe alcoxy, un groupe amino ou un groupe $-NH-CO-NH-R^7$, $R^7$ représentant un atome d'hydrogène ou un groupe alkyle éventuellement substitué par un groupe cyano, par un groupe alcoxy ou par un groupe alcoxycarbonyle, ou encore un groupe cycloalkyle, et
$R^6$ représente un groupe alcynyle, un groupe alcoxyalkyle, un groupe halogénalcoxyalkyle, un groupe cyanalcoxyalkyle, un groupe cycloalcoxyalkyle, un groupe alcénoxyalkyle, un groupe alcynoxy-alkyle, un groupe alkylthioalkyle, un groupe alkylcarbonylalkyle, un groupe alcoxycarbonylalkyle, un groupe alkylthiocarbonylalkyle, de même qu'un groupe aminocarbonylalkyle, le groupe amino pouvant être substitué par des groupes alkyle qui, ensemble avec l'atome d'azote, peuvent également former un noyau hétérocyclique, ainsi qu'un groupe hétérocyclylalkyle pentagonal ou hexagonal relié par C ou N et pouvant contenir jusqu'à 3 atomes d'azote, jusqu'à 2 atomes d'oxygène ou jusqu'à 2 atomes d'azote et 1 atome d'oxygène, un groupe bicyclohétérocyclylalkyle contenant jusqu'à 3 atomes d'azote, les groupes hétérocycliques pouvant être substitués par des groupes alkyle ou par le groupe céto, ainsi qu'un groupe furanone hydrogéné pouvant être substitué par des groupes alkyle, ou un radical de structure

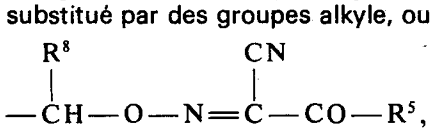

$R^5$ ayant la signification indiquée ci-dessus et $R^8$ représentant un atome d'hydrogène, un groupe méthyle ou un groupe éthyle.

caractérisé en ce que:

a) on fait réagir un acétanilide substitué de formule (II):

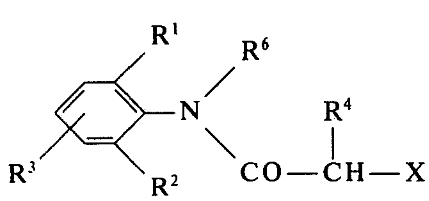

(II)

dans laquelle

$R^1$, $R^2$, $R^3$, $R^4$ et $R^6$ ont les significations indiquées ci-dessus, et
X représente un atome de chlore, un atome de brome, un atome d'iode ou un groupe sulfonyloxy,

avec un dérivé d'acide 2-cyano-2-oximino-acétique de formule (III):

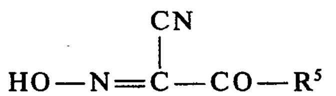
(III)

dans laquelle

$R^5$ a la signification indiquée ci-dessus, éventuellement dans un diluant sous forme de sels alcalins ou alcalino-terreux ou en présence d'un accepteur de protons, ou

b) dans le cas où $R^5$ représente $-NH-CO-NHR^9$ et que

$R^9$ représente un groupe alkyle éventuellement substitué par un groupe cyano, par un groupe alcoxy ou par un groupe alcoxycarbonyle, ou encore un groupe cycloalkyle, mais que
$R^6$ ne représente pas

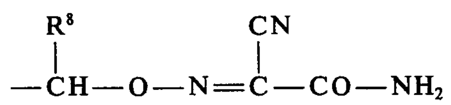

ou

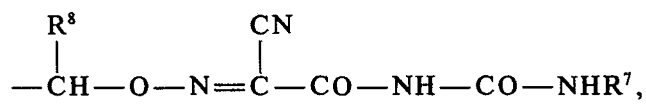

on fait réagir des composés de formule (IV):

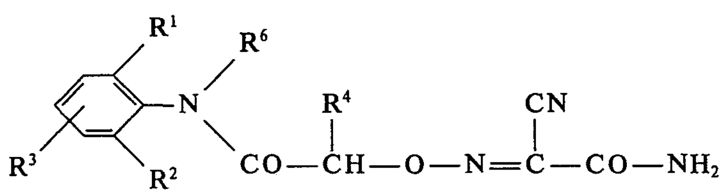
(IV)

dans laquelle

$R^1$, $R^2$, $R^3$ et $R^4$ ont les significations indiquées ci-dessus,
$R^6$ a la signification indiquée ci-dessus, mais ne représente pas

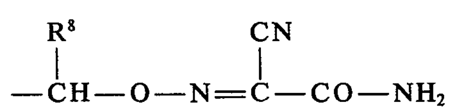

ou

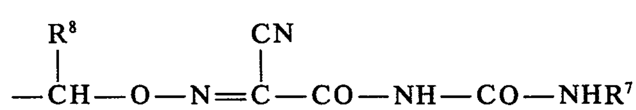

en présence d'une base, avec un isocyanate de formule (V):

$OCN-R^9$ (V)

dans laquelle

$R^9$ a la signification indiquée ci-dessus, éventuellement en présence d'un diluant, ou

c) dans le cas où $R^6$ représente

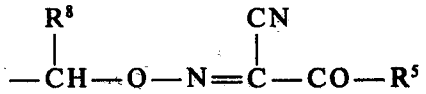

et que $R^5$ est identique dans les deux radicaux, on fait réagir des composés de formule (VI):

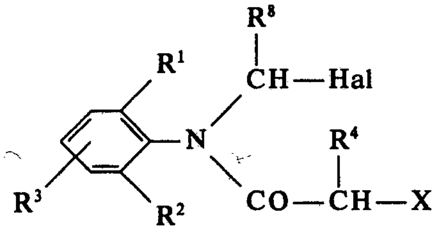

(VI)

dans laquelle

$R^1$, $R^2$, $R^3$, $R^4$, $R^8$ et X ont les significations indiquées ci-dessus, et
Hal représente un atome d'halogène, de préférence, un atome de chlore,

avec 2 moles d'un dérivé d'acide 2-cyano-2-oximinoacétique de formule (III), éventuellement en présence d'un diluant sous forme de sels alcalins ou alcalinoterreux ou en présence d'un accepteur de protons.

3. Parasiticides, caractérisés en ce qu'ils contiennent au moins un oximino-acétanilide substitué de formule (I) suivant les revendications 1 et 2.
4. Utilisation d'oximino-acétanilides substitués de formule (I) suivant les revendications 1 et 2 pour combattre les parasites.
5. Procédé en vue de combattre les parasites, caractérisé en ce qu'on fait agir des oximinoacétanilides substitués de formule (I) suivant les revendications 1 et 2 sur des parasites et/ou leur biotope.
6. Procédé de préparation de parasiticides, caractérisé en ce qu'on mélange des oximino-acétanilides substitués de formule (I) suivant les revendications 1 et 2 avec des diluants et/ou des agents tensio-actifs.